(19) Europäisches Patentamt
European Patent Office
Office européen des brevets

(11) EP 4 239 050 A1

(12) EUROPEAN PATENT APPLICATION
published in accordance with Art. 153(4) EPC

(43) Date of publication:
**06.09.2023 Bulletin 2023/36**

(21) Application number: **21886185.4**

(22) Date of filing: **26.10.2021**

(51) International Patent Classification (IPC):
***C12M 1/00*** *(2006.01)* ***A01N 1/00*** *(2006.01)*
***B01D 71/14*** *(2006.01)* ***B01D 71/16*** *(2006.01)*
***C12N 1/04*** *(2006.01)* ***C12N 5/073*** *(2010.01)*
***C12N 5/075*** *(2010.01)* ***D01F 2/28*** *(2006.01)*

(52) Cooperative Patent Classification (CPC):
**A01N 1/00; B01D 71/14; B01D 71/16; C12M 1/00; C12N 1/04; C12N 5/06; D01F 2/28**

(86) International application number:
**PCT/JP2021/039439**

(87) International publication number:
**WO 2022/092067 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.10.2020 JP 2020182627**

(71) Applicant: **TOYOBO CO., LTD.**
**Osaka-shi**
**Osaka 5300001 (JP)**

(72) Inventors:
- **TAJIMA, Takatsugu**
  **Osaka-shi, Osaka 530-8230 (JP)**
- **TAKII, Yoshinori**
  **Otsu-shi, Shiga 520-0292 (JP)**

(74) Representative: **Vossius & Partner**
**Patentanwälte Rechtsanwälte mbB**
**Siebertstraße 3**
**81675 München (DE)**

# (54) CELL CRYOPRESERVATION HOLLOW FIBER MEMBRANE

(57) In a cell cryopreservation hollow fiber membrane made of a cellulose ester, a decrease in strength during the use for cell cryopreservation is suppressed. A cell cryopreservation hollow fiber membrane, comprising a cellulose ester, wherein the breaking strength in thawing the cell cryopreservation hollow fiber membrane after freezing by vitrification freezing is 80% or more of the breaking strength during wetting before the freezing.

FIG.3

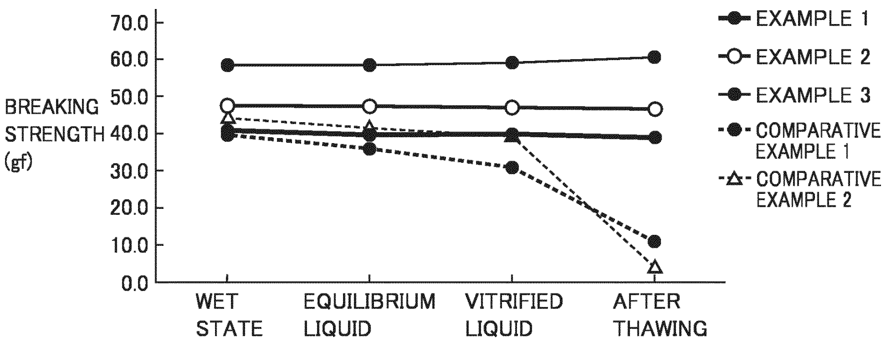


EP 4 239 050 A1

## Description

TECHNICAL FIELD

The present invention relates to a cell cryopreservation hollow fiber membrane.

BACKGROUND ART

Vitrification freezing is known as a method for cryopreserving cells such as egg cells and germ cells. In vitrification freezing, an implement called a Cryotop(R) is used. A Cryotop is an exclusive implement in which a very thin rectangular sheet is attached to a tip of a grip. Cells are cryopreserved using liquid nitrogen or the like with the cells mounted with liquid for freezing such as vitrified liquid on a sheet on the tip of a Cryotop.

In such cell cryopreservation, a method using a hollow fiber membrane (a hollow fiber cryopreservation method) has also been examined in recent years. In the hollow fiber cryopreservation method, cells are frozen and preserved with the cells stored inside a hollow fiber membrane constituted of a cellulose acetate. In this method, the cell viability after the freezing is expected to be improved.

However, a hollow fiber membrane was a relatively fragile material, and may have been damaged during the use thereof for cryopreservation, and had a problem with the handleability. For example, PTL 1 (Japanese Patent No. 5252556), PTL 2 (Japanese Patent No. 5051716), and PTL 3 (Japanese Patent No. 6667903) disclose implements for supporting hollow fibers and the like for improving the handleability of hollow fibers during the cryopreservation.

For example, as conventional hollow fiber membranes, hollow fiber membranes are known that are made of cellulose acetate used for hemodialysis, hemodialysis filtration, or the like as disclosed in PTL 4 (Japanese Patent No. 5440332) and PTL 5 (Japanese Patent No. 5212837).

CITATION LIST

PATENT LITERATURE

PTL 1: Japanese Patent No. 5252556
PTL2: Japanese Patent No. 5051716
PTL 3: Japanese Patent No. 6667903
PTL 4: Japanese Patent No. 5440332
PTL 5: Japanese Patent No. 5212837

SUMMARY OF INVENTION

TECHNICAL PROBLEM

As mentioned above, the exposure to very low temperature as used for cell cryopreservation, the volume change during freezing and subsequent thawing, and the like may have reduced the strength of hollow fiber membranes made of a cellulose ester such as a cellulose acetate to damage the hollow fiber membrane.

Therefore, an object of the present invention is to suppress a decrease in strength in a cell cryopreservation hollow fiber membrane made of a cellulose ester when the hollow fiber membrane is used for cell cryopreservation.

SOLUTION TO PROBLEM

(1) A cell cryopreservation hollow fiber membrane, comprising a cellulose ester,
wherein breaking strength in thawing the cell cryopreservation hollow fiber membrane after freezing by vitrification freezing is 80% or more of breaking strength during wetting before the freezing.
(2) The hollow fiber membrane according to (1), wherein the hollow fiber membrane comprises a nonuniform structure in a thickness direction.
(3) The hollow fiber membrane according to (1) or (2), wherein an average pore size of an outer surface of the hollow fiber membrane is 1.1 or more times as large as an average pore size of an inner surface.
(4) The hollow fiber membrane according to any one of (1) to (3), wherein an average area percentage in a thickness direction cross section is 40% or more and 70% or less.

(5) The hollow fiber membrane according to any one of (1) to (4), wherein a variation in an area percentage of the thickness direction cross section is less than 5% in the thickness direction.

(6) The hollow fiber membrane according to any one of (1) to (3), wherein an arithmetic average roughness of the inner surface of the hollow fiber membrane is 20 nm or less, as measured by atomic force microscopy.

ADVANTAGEOUS EFFECTS OF INVENTION

According to the present invention, in a cell cryopreservation hollow fiber membrane made of a cellulose ester, a decrease in strength during the use for cell cryopreservation can be suppressed.

BRIEF DESCRIPTION OF DRAWINGS

Fig. 1 is a graph showing the results of the measurement of the internal diameters of hollow fiber membranes.
Fig. 2 is a graph showing the results of the measurement of the film thicknesses of the hollow fiber membranes.
Fig. 3 is a graph showing the results of the measurement of the breaking strengths of the hollow fiber membranes.
Fig. 4 is a graph showing the results of the measurement of the breaking elongations of the hollow fiber membranes.
Fig. 5 is a graph showing the results of the measurement of the yield strengths of the hollow fiber membranes.
Fig. 6 is a schematic diagram for describing an example of a method for manufacturing a hollow fiber membrane.

DESCRIPTION OF EMBODIMENTS

Although embodiments of the present invention will be described hereinafter, the present invention is not limited to these. The expression in the form of "A to B" used herein means that the upper limit and the lower limit of the range (namely A or more and B or less). When no unit is described beside A, and a unit is described beside only B, the unit of A is the same as the unit of B.

<Hollow fiber membrane>

A hollow fiber membrane of the present embodiment can be preferably used for cell cryopreservation.

Examples of target cells include pluripotent stem cells including egg cells (fertilized eggs and the like), germ cells, iPS cells, and ES cells, and the like and artificial tissues (cell conglomerates) such as organoids derived from pluripotent stem cells. The cell conglomerates may be constituted of a plurality of cell types. The cells are cryopreserved with the cells disposed in the hollow fiber membrane. A cell suspension containing separate cells may be cryopreserved in the hollow fiber membrane.

(Cellulose ester)

The hollow fiber membrane of the present embodiment contains a cellulose ester. The proportion of the cellulose ester in materials constituting the hollow fiber membrane is preferably 90% by mass or more, more preferably 95% by mass or more, and further preferably 98% by mass or more. The hollow fiber membrane may be constituted of only the cellulose ester.

It is preferable that the hollow fiber membrane has so transparent that cells stored therein can be seen. Since the cellulose ester is a material having high transparency, it is preferable that the proportion of the cellulose ester in the materials constituting the hollow fiber membrane be high.

It is preferable that the hollow fiber membrane be not dissolved in vitrified liquid, or a resin component be not eluted in the vitrified liquid. It is also preferable in this viewpoint that the proportion of the cellulose ester in the materials constituting the hollow fiber membrane be high.

Examples of the cellulose ester includes cellulose acetate, cellulose phthalate, and cellulose succinate. The cellulose ester is preferably cellulose acetate. Cellulose acetate has resistance to chlorine as a sterilizer, and can be sterilized with chlorine.

Example of the cellulose acetate includes cellulose triacetate, cellulose monoacetate, cellulose diacetate, cellulose acetate butyrate, and cellulose acetate propionate. The cellulose acetate is preferably cellulose triacetate from the viewpoint of durability and the like.

For example, as cellulose acetates, various cellulose acetates (L-20, 30, 40, 50, and 70, LT-35, 55, and 105, and the like) that vary in the acetylation degree, the polymerization degree, and the like are marketed from Daicel Corporation. It is preferable to use cellulose acetate wherein the 6% viscosity is a relatively low viscosity of more than 140 mPa·s and less than 200 mPa·s (relatively low-viscosity polymer).

The acetylation degree of the cellulose acetate is preferably 53 to 62%, more preferably, 55 to 61.5%, and further preferably 58 to 61.5%. The acetylation degree indicates the degree at which acetic acid groups are substituted for hydroxyl groups in cellulose. Although the theoretical upper limit of the acetylation degree is 62.5%, too high an acetylation degree may reduce the solubility and the formability.

(Strength and elongation such as breaking strength)

In the hollow fiber membrane of the present embodiment, the breaking strength in thawing the hollow fiber membrane after the freezing by vitrification freezing is 80% or more of the breaking strength during wetting before the freezing, and is preferably 90% or more thereof, and more preferably 95% or more thereof.

In the hollow fiber membrane of the present embodiment, the breaking elongation in thawing the hollow fiber membrane after the freezing by vitrification freezing is preferably 80% or more of the breaking elongation during wetting before the freezing, and is more preferably 90% or more thereof, and further preferably 95% or more thereof.

In the hollow fiber membrane of the present embodiment, the yield strength in thawing the hollow fiber membrane after the freezing by vitrification freezing is preferably 80% or more of the yield strength during wetting before the freezing, and is more preferably 90% or more thereof, and further preferably 95% or more thereof.

In the hollow fiber membrane containing the cellulose ester of the present embodiment, a decrease in strength in using the hollow fiber membrane for cellular cryopreservation is thus suppressed.

The breaking strength, breaking elongation and yield strength are measured by a method for measuring the strength and elongation (breaking strength, breaking elongation, and yield strength) in the Examples described below.

The vitrification freezing is a method in which immersing cells in liquid nitrogen or the like to rapidly lower the temperature freezes the cells in an amorphous glass state while preventing water crystallization, which is said to easily occur at -60°C to -15°C. This method is more excellent than slow freezing in that ice crystal formation does not damage cells, it takes a short time for the treatment, special equipment is unnecessary, and cells are satisfactorily preserved for a long period. Many specific methods for cell vitrification freezing have been developed, and examples thereof include a method for immersing cells in a freezing-resistant preservative medium to rapidly freeze the cells in liquid nitrogen or in an ultralow temperature freezer at -80°C or less, more preferably -190°C or less.

The conditions for the vitrification freezing when the above-mentioned strength and elongation (breaking strength, breaking elongation and yield strength) before and after the freezing and the thawing are compared are as follows.

[Conditions of the vitrification freezing]

(Equilibrium liquid)

Composition: Ethylene glycol (7.5% by mass), dimethyl sulfoxide (7.5% by mass), and water
Immersion time: 4 minutes
(Vitrified liquid)
Composition: Ethylene glycol (15% by mass), dimethyl sulfoxide (15% by mass), sucrose (0.5 M in the vitrified liquid), and water
Immersion time: 30 seconds

(Cryopreservation conditions)

Freezing: The cells are immersed in liquid nitrogen ($LN_2$).
Preservation: The cells are preserved for a week while the cells remain immersed in liquid nitrogen ($LN_2$).

(Thawing)

The cells are immersed in a thawing liquid (aqueous 1 M sucrose solution) for 1 minute.

The cells are immersed in a diluent (aqueous 0.5 M sucrose solution) for 3 minutes.

The cells are immersed in washing liquid (TCM199 medium, which is commercially available (Thermo Fisher Scientific K.K.)), for 5 minutes and further immersed in different washing liquid having the same composition for 5 minutes.

(Shapes and the like of hollow fiber membrane)

The inner diameter of the hollow fiber membrane is preferably 30 μm or more and 300 μm or less and more preferably 35 μm or more and 260 μm or less.

The thickness of the hollow fiber membrane is preferably 20 to 200 μm and more preferably 30 to 150 μm. The film thickness can be calculated from "(outer diameter - inner diameter)/2".

It is preferable that the hollow fiber membrane (hollow fiber type membrane) be constituted of a semipermeable membrane. Although the cells stored and cryopreserved in the hollow fiber membrane are blocked for passage, a culture solution, a cryopreservative medium, a cryoprotectant contained the preservative medium, or the like is passed. The state in which the cells are therefore encapsulated in the internal space of the hollow fiber membrane can be maintained. That is, the hollow fiber membrane has an advantage of enabling easy replacement of the culture solution, the intracellular fluid, and the like in the internal space with a cryopreservative medium.

The hollow percentage of the hollow fiber membrane is preferably 10 to 65% and more preferably 12 to 55%. The hollow percentage is the areal proportion of hollow portions in the cross section of the hollow fiber membrane, and is represented by "Hollow portion cross-sectional area/(Membrane portion cross-sectional area + Hollow portion cross-sectional area) × 100 (%)".

The average pore size of the hollow fiber membrane (average pore size of the micropores in the whole membrane) is preferably 10 μm or less. Examples of the method for measuring the average pore size include the bubble point method and mercury porosimetry.

The hollow fiber membrane of the present embodiment is preferably a membrane having a nonuniform structure in the thickness direction (asymmetric structure). It is believed that when the hollow fiber membrane has the asymmetric structure, the hollow fiber membrane has a high effect of suppressing a decrease in strength of the hollow fiber membrane due to very low temperature to be used for cell cryopreservation, volume change in the freezing and the subsequent thawing, and the like. Although the reason therefor is unclear, it is considered as one reason that the buffer capability of polymer chains in the nonuniform structure is higher than that in a uniform structure, and the volume change and the like of the hollow fiber membrane more hardly affects the nonuniform structure than the uniform structure.

Examples of the hollow fiber membrane having an asymmetric structure include a hollow fiber membrane that varies in density (porosity and the cross-sectional open area percentage) and the like in the thickness direction. An example of such a hollow fiber membrane is a membrane wherein the membrane has a fine layer on one surface side, this fine layer functions as a separation active layer that defines the pore size of the hollow fiber membrane substantially, and the density of the other surface side is lower than that of the fine layer.

In the hollow fiber membrane having the asymmetric structure, the open area percentage of one surface is preferably different from the open area percentage of the other surface. The open area percentage of the one surface having a higher open area percentage is preferably 1.1 times or more, more preferably 1.3 times or more, as large as the open area percentage of the other surface. It is believed that when the hollow fiber membrane has such an asymmetric structure, heat is rapidly transmitted from a solution outside the hollow fiber membrane to a solution inside the hollow fiber membrane in vitrification freezing, the generation of ice crystals can therefore be suppressed, and damage to the membrane structure and the cells are reduced.

In the measurement of the open area percentage of the membrane surface, the hollow fiber membrane is first imaged using a scanning electron microscope (SEM) of 10,000 times. A region of 762 pixels in length × 620 pixels in width is cut out of the obtained image, the image is then binarized into white/black using image analysis software (for example, WinROOF2013), and the open area percentages of the inner surface and the outer surface of the hollow fiber membrane are calculated. Ten visual fields are subjected to this, and the average thereof is calculated and defined as the surface open area percentage.

The area percentage of the hollow fiber membrane is an area proportion of solid portions (portions in which the membrane is present) except hollow portions in the thickness direction cross section (transverse section) of the hollow fiber membrane. The image obtained by photographing the cross section of the hollow fiber membrane is analyzed with an SEM to determine the area percentage. The membrane cross section is specifically divided into three equal regions in the film thickness direction to measure the respective area percentages. The three regions are a region A including the outer surface, a region B including the inner surface, a region between the region A and the region B (central area C). The magnification of the SEM only has to be a magnification that enables recognizing hollow portions and solid portions, and, for example, 5000 times to 20000 times are suitable for the measurement of the hollow fiber membrane of the present invention. The area percentage is calculated by a method using image analysis. Hollow portions and solid portions (polymer portions) are specifically subjected to binarization processing using image analysis software (for example, WinROOF2013). After the binarization processing, the area percentage is calculated from the proportion between the total area of the hollow portions and the total area of the polymer portions.

In the present embodiment, the hollow fiber membrane is characterized in that while the hollow fiber membrane has the above-mentioned asymmetric structure, the bulk density (area percentage in the thickness direction cross section) hardly varies (and is preferably almost constant) in the membrane thickness direction. Although the reason therefor is unclear, it is believed that since the hollow fiber membrane has the above-mentioned structural characteristics, the hollow fiber membrane can resist rapid temperature change and the volume change of the vitrified liquid. It is believed that the embrittlement or destruction of the hollow fiber membrane structure due to the freezing and thawing can therefore be suppressed, and the membrane strength can be maintained.

The phrase hardly vary in the area percentage in the thickness direction cross section means, for example, that there is a small difference in the area percentage (%) among the region A including the outer surface, the region B including the inner surface, and the central area C (region between the region A and the region B) in the thickness direction cross section. Specifically, when the area percentages of the regions A, B, and C are measured, the absolute value of the difference between any two area percentages selected from a (the area percentage of the region A), b (the area percentage of the region B), and c (the area percentage of the region C) is preferably less than 5% and more preferably less than 3% (refer to the following expression). For example, the region A is a region spreading from the outer surface to a line deeper than the outer surface by 30% of the membrane thickness, and the region B is a region spreading from the inner surface to a line deeper than the inner surface by 30% of the membrane thickness.

Preferably,

$$|a - b| < 5\%,\ |b - c| < 5\%,\ \text{and}\ |c - a| < 5\%$$

More preferably,

$$|a - b| < 3\%,\ |b - c| < 3\%,\ \text{and}\ |c - a| < 3\%$$

The average area percentage of the thickness direction cross section (for example, "(a + b + c)/3") is preferably 40% or more and 70% or less (refer to the following expression).

$$40\% \leq (a + b + c)/3 \leq 70\%$$

In the present invention, it is preferable that the hollow fiber membrane has high smoothness of the inner surface. Even though egg cells and germ cells are in contact with the inner surface of the hollow fiber membrane, the high smoothness of the inner surface enables minimizing a risk of damaging the cell surfaces and the like. Here, high smoothness means that the arithmetic average roughness Ra value is 20 nm or less. As the smoothness becomes higher, and damage to the cells is reduced. Therefore, the Ra value is more preferably 10 nm or less and further preferably 1 nm or more and less than 8 nm. The arithmetic average roughness Ra value can be measured using an atomic force microscope (AFM).

<Method for manufacturing hollow fiber membrane>

The present invention also relates to a method for manufacturing the hollow fiber membrane containing the above-mentioned cellulose ester.

For example, the method for manufacturing the hollow fiber membrane of the present embodiment is the following method.

A method for manufacturing the above-mentioned hollow fiber membrane, comprising:

a spinning step of discharging spinning dope and internal liquid from a double tubular nozzle through an aerial traveling portion to coagulation liquid, coagulating the spinning dope in the coagulation liquid, and drawing a coagulated material of the spinning dope out of the coagulation liquid to obtain a hollow fiber membrane,
wherein the spinning dope comprises a resin raw material containing a cellulose ester, a solvent, and a non-solvent,
the internal liquid comprises water,
the temperature of the spinning dope in the nozzle is 70 to 110°C, the temperature of the internal liquid is 40 to 70°C,
the proportion in amount of the solvent to the non-solvent in the coagulation liquid is 60/40 to 80/20, and
the nozzle draft ratio is 0.4 to 0.9.

The concentration of the cellulose ester in the spinning dope is preferably 10 to 30% by mass.

In the spinning dope, it is preferable that the proportion of the amount of the solvent to the total amount of the solvent and the non-solvent be 60 to 80% by mass.
The solvent is preferably an aprotic polar solvent.
The non-solvent is preferably a glycol ester.
The direct distance of the aerial traveling portion is preferably 10 to 50 mm.

[Spinning step]

With reference to Fig. 6, in the spinning step, spinning dope 10a and internal liquid 10b are discharged from double tubular nozzle 11 through an aerial traveling portion (air gap) 20 to coagulation liquid 21, the spinning dope is coagulated in coagulation liquid 21, and a coagulated material of the spinning dope is drawn out of coagulation liquid 21 to obtain a hollow fiber membrane 16. For example, the hollow fiber membrane is drawn out with a guide in the liquid 12 and rollers 13, 14, and 15.
Nozzle 11 is in a double tubular shape, and comprises an outer tube and an inner tube provided in the outer tube. The spinning dope is discharged from a gap (slit) between the outer tube and the inner tube, and the internal liquid is discharged from inside the inner tube. The proportion of the diameter of the inner tube (inner diameter of the slit) to the diameter of the outer tube (outer diameter of the slit) is preferably 110 to 300% and more preferably 110 to 200%. The diameter of the outer tube is preferably 220 to 400 μm and more preferably 220 to 330 μm. The diameter of the inner tube is preferably 150 to 330 μm and more preferably 150 to 270 μm. The diameter of the inner tube is preferably equivalent to the diameter of the hollow fiber membrane.
The proportion of the cross-sectional area of the inner tube to the cross-sectional area of the slit is preferably 80 to 120%. Since the discharge linear speed of the membrane-forming stock solution can be equivalent to the drawing speed by adopting such a nozzle, the interfacial friction between the discharged membrane-forming stock solution and the internal liquid can be reduced, and the inner surface of the hollow fiber membrane can be prevented from being rough.
The discharge linear speed of the membrane-forming stock solution is calculated by dividing the discharge volume of the membrane-forming stock solution by the slit cross-sectional area [$\pi(a/2)^2 - \pi(b/2)^2$] calculated from the nozzle slit outer diameter (a) and the nozzle slit inner diameter (b) (refer to the following expression).
Discharge linear speed of membrane-forming stock solution [m/minute] = Discharge volume of membrane-forming stock solution/slit cross sectional area
The drawing speed is the rotational speed (surface speed) of roller 13 provided at the outlet of the coagulation bath (refer to Fig. 6).
The nozzle draft ratio, which is the proportion of the discharge linear speed to the drawing speed (drawing speed/discharge linear speed), is 0.4 to 0.9 and preferably 0.5 to 0.9. Thus, an increase in the discharge linear speed relative to the drawing speed of the membrane-forming stock solution enables obtaining a characteristic membrane structure of the hollow fiber of the present invention.
The direct distance of aerial traveling portion 20 (distance between the tip of nozzle 11 and the liquid surface of coagulation liquid 21) is preferably 10 to 50 mm and more preferably 10 to 40 mm.
The hollow fiber membrane obtained by the spinning step may be further subjected to a step of washing with pure water (water washing step). The flow of water in the water washing step is preferably a flow in the direction opposite to the moving direction of the hollow fiber membrane (counter flow), but may be a flow in the same direction as the moving direction of the hollow fiber membrane (parallel flow).

(Spinning dope)

Spinning dope 10a contains the above-mentioned resin raw material containing the cellulose ester, the solvent, and the non-solvent.
The temperature (preset temperature) of the spinning dope in nozzle 11 is 70 to 110°C and preferably 70 to 100°C.
The concentration of the cellulose ester in the spinning dope is preferably 10 to 30% by mass and more preferably 10 to 25% by mass. When the concentration of the cellulose ester is too low, the strength of the hollow fiber membrane decreases. Meanwhile, when the concentration of the cellulose ester is too high, the viscosity of the spinning dope is too high, and the spinning may be difficult.
The solvent is liquid that can dissolve the cellulose ester. The solvent is preferably a polar solvent, and is preferably soluble in water. The polar solvent is preferably an aprotic polar solvent. Examples of the aprotic polar solvent include N-methylpyrrolidone (NMP), dimethylformamide (DMF), dimethylacetamide (DMA), dimethyl sulfoxide (DMSO), and acetonitrile.
The non-solvent is liquid that does not dissolve the cellulose ester (except water). Examples of the non-solvent include glycol esters, glycerin, and alcohols, but the non-solvent is preferably a glycol ester. Examples of the glycol ester include ethylene glycol, triethylene glycol (TEG), polyethylene glycol (polyethylene glycol 200, polyethylene glycol 400, and the like), and propylene glycol.

In the spinning dope, the proportion in amount of the solvent (S) to the non-solvent (NS) (SINS ratio) is 60/40 to 80/20, and more preferably 65/35 to 75/25. If the S/NS ratio in the spinning dope is too low, the cellulose ester is unstably dissolved. Therefore, the spinning stability is deteriorated, or the asymmetric structure suitable for use of the present invention may not be obtained. If the S/NS ratio increases, the hollow fiber membrane having the asymmetric structure is not obtained, or the spinning stability may be deteriorated.

The spinning dope may further contain water in addition to the solvent and the non-solvent.

The addition order and the mixing method of materials in mixing the resin raw material containing the cellulose ester to be used as a component of the hollow fiber membrane, the solvent, and the non-solvent are not particularly limited.

(Internal liquid)

The internal liquid contains water. The water content in the internal liquid is 95 to 100% by mass and preferably 98 to 100% by mass.

The inner surface of the hollow fiber membrane is preferably highly smooth. It is because damage due to the contact with the cells to be cryopreserved can be reduced.

It is preferable that the spinning dope be discharged from the nozzle, the inner surface be then rapidly coagulated (fixed) before disturbance influence, and the phase separation be not excessively advanced to enhance the smoothness of the inner surface of the hollow fiber membrane. It is preferable to suppress a change in the internal diameter and the like after the hollow fiber membrane structure is fixed as much as possible without applying external force such as stretching to the inner surface during and after the coagulation.

It is preferable that internal liquid 10b highly coagulable to spinning dope 10a be used, or a spinning dope composition or temperature conditions for coagulating spinning dope 10a easily be adopted in order to coagulate the inner surface rapidly.

In the manufacturing method of the present embodiment, internal liquid containing water, which is highly coagulable to the spinning dope containing the cellulose ester, is therefore used.

Ethylene glycol, triethylene glycol, polyethylene glycol 200 or 400, glycerin, the propylene glycol, and the like, which are commonly used as a non-solvent for the cellulose ester besides water, can be used alone or as a mixture thereof. When internal liquid mainly containing water is used, as a component other than water, the non-solvent or up to 5% by weight of a solvent for cellulose triacetate-based polymer, such as N-methylpyrrolidone, dimethylacetamide, dimethylformamide, or dimethyl sulfoxide, can be added.

The temperature (preset temperature) of the internal liquid in nozzle 11 is 40 to 70°C and preferably 45 to 65°C. As described above, the temperature (preset temperature) of the spinning dope in nozzle 11 is 70 to 110°C, but it is preferable that the temperature of the internal liquid be set at a temperature lower than this. The temperature of the internal liquid in nozzle 11 is lower than the temperature of the spinning dope in nozzle 11 by preferably 10°C or more, more preferably 20°C or more, and further preferably 30°C or more.

It is preferable to set a temperature difference between the spinning dope and the internal liquid for enhancing the coagulability (coagulation speed) of the inner surface of the spinning dope (hollow fiber membrane) in discharging spinning dope 10a and internal liquid 10b from double tubular nozzle 11. The enhancement of coagulation speed of the inner surface enables obtaining a hollow fiber membrane having an asymmetric structure (nonuniform structure in the thickness direction). The smoothness of the hollow fiber membrane inner surface can be enhanced to reduce a change in the bulk density (area percentage) in the membrane thickness direction in the membrane cross section in spite of the asymmetric structure partly due to setting the temperature of the internal liquid in a specific range, and then adjusting the composition of the coagulation liquid described below to a specific range or adjusting the nozzle draft. It is preferable to use a nozzle having a structure that can control the temperature of the spinning dope and the temperature of the internal liquid separately until directly before the discharge to set a temperature difference between the spinning dope and the internal liquid.

(Coagulation liquid)

The coagulation liquid preferably contains a solvent and a non-solvent (except water). In this case, the coagulation liquid may further contain water in addition to the solvent and the non-solvent.

The proportion of the total amount of the solvent and the non-solvent in the coagulation liquid (concentration of the coagulation liquid) is 60 to 90% by mass and preferably 65 to 90% by mass. Therefore, the characteristic structure of the hollow fiber membrane of the present invention can be obtained.

The temperature of the coagulation liquid is preferably 20 to 60°C and more preferably 30 to 50°C.

EXAMPLES

Although the present invention will be described in more detail hereinafter by giving the Examples, the present invention is not limited to these.

[Example 1]

The hollow fiber membrane of Example 1 was manufactured under the following conditions by the method for manufacturing the hollow fiber membrane described in the embodiments.

(Composition of spinning dope)

Raw resin (cellulose ester): Cellulose triacetate (CTA) (LT75, produced by Daicel Corporation)
Raw resin concentration (polymer concentration): 17.5% by mass (in spinning dope)
Solvent: N-methylpyrrolidone (NMP)
Non-solvent: Triethylene glycol (TEG)
[Solvent/non-solvent (SINS) ratio = 7/3]
Internal liquid: Water

(Preparation of spinning dope)

Powder of the above-mentioned raw resin was mixed with the other materials to prepare spinning dope to be used in the spinning step.

(Composition of coagulation liquid)

Solvent (S): NMP
Non-solvent (NS): TEG
Water
Concentration of coagulation liquid [(mass of S + mass of NS)/mass of coagulation liquid]: 78% by mass
The S/NS ratio is the same as that of the spinning dope.

(Conditions of spinning step)

Discharge temperature (preset temperature) of spinning dope: 93°C
Discharge temperature (preset temperature) of internal liquid (water): 55°C
Nozzle: Double tubular nozzle (diameter of outer tube: 270 μm, diameter of inner tube: 200 μm)
Distance of aerial traveling portion (air gap length): 25 mm,
Residence time of aerial traveling portion: 0.025 seconds
Temperature of coagulation liquid: 43°C
Drawing speed: 60 m/minute
Nozzle draft ratio: 0.78

[Conditions for water washing step]

Flow in water washing tank: counter flow

Temperature 98°C

[Examples 2 and 3]

As shown in Table 1, the nozzle draft ratio was changed. The hollow fiber membranes of Example 2 and Example 3 were manufactured in the same way as in Example 1 except this point. Table 2 showed the results of measuring the arithmetic average roughnesses and area percentages of the inner surfaces of the obtained hollow fiber membranes.

[Comparative Examples 1 and 2]

As shown in Table 1, the compositions of the spinning dope, the internal liquid, and the coagulation liquid and each of the manufacturing conditions were changed. Liquid paraffin was used as the internal liquid. The preset temperature of the internal liquid was not particularly controlled, and is equivalent to the temperature of the spinning dope. The hollow fiber membranes of Comparative Example 1 and Comparative Example 2 were manufactured in the same way as in Example 1 except these points.

[Table 1]

| | Example 1 | Example 2 | Example 3 | Comparative Example 1 | Comparative Example 2 |
|---|---|---|---|---|---|
| Spinning dope polymer concentration (% by mass) | 17.5 | 17.5 | 17.5 | 18 | 20 |
| Ratio of NMP/TEG in spinning dope | 7/3 | 7/3 | 7/3 | 7/3 | 7/3 |
| Outer diameter of nozzle slit (μm) | 270 | 270 | 270 | 1000 | 1000 |
| Inner diameter of nozzle slit (μm) | 200 | 200 | 200 | 810 | 810 |
| Preset temperature of spinning dope (°C) | 93 | 93 | 93 | 103 | 125 |
| Preset temperature of internal liquid (°C) | 55 | 55 | 55 | Not controlled | Not controlled |
| Internal liquid | Water | Water | Water | Liquid paraffin | Liquid paraffin |
| Air gap length(mm) | 25 | 25 | 25 | 47 | 58 |
| Coagulation liquid concentration (%) | 78 | 78 | 78 | 15 | 15 |
| Temperature of coagulation liquid (°C) | 43 | 43 | 43 | 24 | 31 |
| Nozzle draft ratio | 0.77 | 0.69 | 0.58 | 15.2 | 15.6 |
| Flow in water wash tank | Counter flow | Counter flow | Counter flow | Parallel flow | Counter flow |

[Measurement of area percentage]

Each of the hollow fiber membranes in a wet state was immersed in liquid nitrogen for freezing, then taken out of the liquid nitrogen, and immediately bent for fracture to obtain a sample having a smooth cross section (the thickness direction cross section). The sample was fixed on a sample stand so that the cross section was observed, and the cross section of the sample was subjected to carbon shadowing. The cross section of the sample after the vapor deposition was imaged at an acceleration voltage of 5 kV and a magnification of 10000 using a scanning electron microscope (S-2500, manufactured by Hitachi High-Tech Corporation). The vicinities of the centers of the regions corresponding to the above-mentioned regions A to C (the region A including the outer surface, the region B including the inner surface, the central area C between the region A and the region B) in the obtained image were subjected to the binarization processing of hollow portions and polymer portions using image analysis software WinROOF2013. After the binarization processing, the area percentage was calculated from the proportion between hollow portions and polymer portions. Here, the region A is a region spreading from the outer surface to a line deeper than the outer surface by 30% of the membrane thickness, and the region B is a region spreading from the inner surface to a line deeper than the inner surface by 30% of the membrane thickness.

Since the fracture or an electron beam may have molten a part of the cross section to make the structure unclear, in that case, a portion having a clear structure was used and measured, or a photograph was retaken after sample change.

Table 2 shows the results of the measurement of the area percentage.

[Measurement of arithmetic average roughness]

Each hollow fiber membrane cut obliquely to the longitudinal direction of the hollow fiber membrane so that the inner surface of the hollow fiber membrane was able to be observed was provided as a sample. The sample was observed in the atmosphere in the DFM mode using an atomic force microscope E-Sweep (Hitachi High-Tech Corporation). An Si-DF3 was used as a cantilever, and a 20 μm scanner was used as a scanner. The arithmetic average roughness of the inner surface of the hollow fiber membrane (Ra) was measured with the observation visual field adjusted to a 2-μm square at 256 × 256 pixels. Table 2 shows the result of the measurement of the arithmetic average roughness (Ra).

[Table 2]

| | | Example 1 | Example 2 | Example 3 | Comparative Example 1 | Comparative Example 2 |
|---|---|---|---|---|---|---|
| Area percentage (%) | a | 47 | 50 | 58 | Unmeasurable | Unmeasurable |
| | b | 48 | 51 | 58 | Unmeasurable | Unmeasurable |
| | c | 47 | 53 | 58 | Unmeasurable | Unmeasurable |
| Arithmetic average roughness(nm) | | 4.5 | 4.6 | 4.6 | 8.0 | 9.4 |

<Cryopreservation test>

The hollow fiber membranes of the Examples 1 to 3 and Comparative Examples 1 and 2 were subjected to cryopreservation tests using vitrification freezing. The conditions for the vitrification freezing are the same as the conditions in measuring the breaking strength (breaking strength at the time of the thawing after the freezing by vitrification freezing) described above.

Each of the hollow fiber membranes of the Examples 1 to 3 and Comparative Examples 1 and 2 in the cryopreservation test was subjected to the following measurements in an original dry state ("dry state"), in a state wet with water ("wet state"), a state in which equilibrium liquid permeated ("equilibrium liquid"), a state in which vitrified liquid permeated ("vitrified liquid"), and a state thawed after the freezing ("after thawing").

[Measurement of inner diameter, outer diameter, and film thickness of hollow fiber membrane]

The inner diameter, outer diameter, and film thickness were measured by the following methods.

A suitable number of the hollow fiber membranes are passed through a hole having a diameter of 3 mm and made at the center of a slide glass so that the hollow fiber membranes did not fall through. The hollow fiber membranes are cut along the top and bottom surfaces of the slide glass with a razor to obtain a hollow fiber membrane cross section sample. The obtained hollow fiber membrane cross section sample is measured for the inner diameter and outer diameter of the hollow fiber membrane using a projector (NIKON CORPORATION, PROFILE PROJECTOR V-12).

Specifically, one hollow fiber membrane cross section was measured for the sizes of the hollow fiber membrane outer surface in the X-X direction and the Y-Y direction (two orthogonal directions on the cross section), and the arithmetic average of those values was defined as the outer diameter of the one hollow fiber membrane cross section. One hollow fiber membrane cross section was measured for the sizes of the hollow portion in the X-X direction and the Y-Y direction (two orthogonal directions on the cross section), and the arithmetic average was defined as the inner diameter of the one hollow fiber membrane cross section. Ten cross sections were measured in the same way to define the averages as the inner diameter and the outer diameter.

The film thickness (average) is calculated based on the measurement results of the inner diameter and the outer diameter of the hollow fiber membrane (averages) from the expression "(outer diameter - inner diameter)/2".

Table 3 (Fig. 1) shows the measurement results of the inner diameters of the hollow fiber membranes (averages), and Table 4 (Fig. 2) shows the measurement results of the film thicknesses of the hollow fiber membranes (averages). Since the hollow fiber membrane cross section samples of the Comparative Examples were not able to be manufactured due to low strengths of the hollow fiber membranes after the thawing, the inner diameter and the film thickness were not able to be measured.

[Table 3]

| | | Example 1 | Example 2 | Example 3 | Comparative Example 1 | Comparative Example 2 |
|---|---|---|---|---|---|---|
| Inner diameter (μm) | Dry state | 199.2 | 188.5 | 220.1 | 192.3 | 195.0 |
| | Wet state | 200.1 | 192.3 | 219.5 | 193.1 | 197.1 |
| | Equilibrium liquid | 202.3 | 192.7 | 223.8 | 196.5 | 195.3 |
| | Vitrified liquid | 199.3 | 194.4 | 219.1 | 194.9 | 193.3 |
| | After thawing | 200.4 | 191.9 | 222.2 | Unmeasurable | Unmeasurable |

[Table 4]

| | | Example 1 | Example 2 | Example 3 | Comparative Example 1 | Comparative Example 2 |
|---|---|---|---|---|---|---|
| Film thickness (μm) | Dry state | 25.7 | 32.5 | 38.3 | 16.1 | 16.9 |
| | Wet state | 25.4 | 30.4 | 38.4 | 16.8 | 15.8 |
| | Equilibrium liquid | 25.2 | 31.0 | 36.3 | 14.2 | 16.4 |
| | Vitrified liquid | 25.4 | 28.2 | 36.5 | 14.5 | 16.5 |
| | After thawing | 25.9 | 31.0 | 36.5 | Unmeasurable | Unmeasurable |

[Measurement of strength and elongation]

The hollow fiber membranes of Examples 1 to 3 and Comparative Examples 1 and 2 were measured for the strength and elongation (breaking strength, breaking elongation and yield strength) by the following methods.

The strength and elongation of the hollow fiber membrane was measured using a tensile tester (UTMII, manufactured by Toyo Baldwin Co. Ltd.). One hollow fiber membrane was cut to a length of around 15 cm, and the cut hollow fiber membrane was fixed to chucks (distance: around 10 cm) so that the cut hollow fiber membrane was tightened between the chucks. The hollow fiber membrane was pulled in a temperature humidity environment of 20 ± 5°C and 60 ± 10% Rh at a cross head speed of 10 cm/min.

The load (breaking strength) and the elongation (breaking elongation) per yarn at the breaking point of the hollow fiber membrane and the load (yield strength) and elongation (yield elongation) per yarn at the yield point were read from the obtained S-S curve. The load and the elongation were specifically obtained using the method shown in [0061] in Japanese Patent Laying-Open No. 2011-212638.

Table 5 to Table 7 (Fig. 3 to Fig. 5) show the results of the measurement of the breaking strength, the breaking elongation, and the yield strength, respectively. Each of Examples and Comparative Examples was measured five times, and the averages thereof are shown as measured values.

[Table 5]

| | | Example 1 | Example 2 | Example 3 | Comparative Example 1 | Comparative Example 2 |
|---|---|---|---|---|---|---|
| Breaking strength (gf) | Dry state | 45.6 | 55.9 | 67.9 | 33.0 | 44.1 |
| | Wet state | 40.4 | 47.3 | 58.4 | 39.4 | 44.3 |
| | Equilibrium liquid | 39.6 | 47.3 | 58.4 | 35.8 | 41.6 |
| | Vitrified liquid | 39.8 | 46.9 | 59.1 | 30.8 | 39.3 |
| | After thawing | 38.9 | 46.6 | 60.4 | 10.6 | 3.8 |

[Table 6]

| | | Example 1 | Example 2 | Example 3 | Comparative Example 1 | Comparative Example 2 |
|---|---|---|---|---|---|---|
| Breaking elongation (%) | Dry state | 19.0 | 16.3 | 15.4 | 26.9 | 51.9 |
| | Wet state | 30.4 | 25.5 | 24.5 | 33.8 | 45.6 |
| | Equilibrium liquid | 29.6 | 24.7 | 25.2 | 51.1 | 64.2 |
| | Vitrified liquid | 30.4 | 26.4 | 23.3 | 45.0 | 58.6 |
| | After thawing | 29.4 | 25.7 | 25.9 | 3.4 | 1.5 |

[Table 7]

| | | Example 1 | Example 2 | Example 3 | Comparative Example 1 | Comparative Example 2 |
|---|---|---|---|---|---|---|
| Yield strength (gf) | Dry state | 35.5 | 46.1 | 55.5 | 21.8 | 21.3 |
| | Wet state | 23.9 | 30.3 | 38.1 | 10.2 | 12.1 |
| | Equilibrium liquid | 24.5 | 31.4 | 38.4 | 10.7 | 14.3 |
| | Vitrified liquid | 24.6 | 31.6 | 41.0 | 12.5 | 15.4 |
| | After thawing | 23.3 | 29.9 | 38.3 | <5 | <5 |

As shown in Table 5 (Fig. 3), in each of the hollow fiber membranes of Comparative Examples, the breaking strength markedly decreases in the state after the thawing through the freezing as compared with the values before the freezing ("dry state" "wet state" "equilibrium liquid", and "vitrified liquid" in the Table and the Figure). Meanwhile, in the case of the hollow fiber membranes of Examples, the breaking strength hardly decreases even in the state after the thawing as compared with the values before the freezing. In each of the hollow fiber membranes of Examples, the breaking strength in the thawed state after the freezing by vitrification freezing is 95% or more of the breaking strength at the time of the wetting before the freezing ("wet state").

As shown in Table 6 and Table 7 (Fig. 4 and Fig. 5), in each of the hollow fiber membranes of Comparative Examples, the breaking elongation and the yield strength markedly decrease in the state after the thawing through the freezing as compared with the values before the freezing. Meanwhile, in the case of the hollow fiber membranes of Examples, the breaking elongation and the yield strength hardly decrease even in the state after the thawing as compared with the values before the freezing. In each of the hollow fiber membranes of Examples, the breaking elongation or the yield strength in the thawed state after the freezing by vitrification freezing is 95% or more of the breaking elongation or the yield strength at the time of the wetting before the freezing ("wet state").

These results show that a decrease in strength in using the hollow fiber membranes of Examples for cell cryopreservation can be suppressed.

<Evaluation of cell viability>

Cells (germ cells derived from a pig) were frozen by vitrification freezing (hollow fiber cryopreservation) and thawed using each of the hollow fiber membranes of Examples 1 to 3 and Comparative Example 1. The viable cell count was then measured to calculate the viability (the proportion of the viable cell count to the test cell count).

The cells were frozen by vitrification freezing (the Cryotop method) and thawed using a Cryotop (Cryotop: a registered trademark, manufactured by Kitazato Corporation), which was a commercially available cell cryopreservation implement. The viable cell count was then measured to calculate the viability.

Table 8 shows the evaluation results of the above-mentioned viabilities with the test cell counts (the number of cells tested) and the viable cell counts. The conditions for the vitrification freezing are the same conditions as in measuring the strength and elongation described above. In the hollow fiber membrane cryopreservation, the cells were cryopreserved with the cells stored in the hollow fiber membrane.

[Table 8]

| | Example 1 | Example 2 | Example 3 | Comparative Example 1 | Commercial item |
|---|---|---|---|---|---|
| Test cell count (cell) | 34 | 33 | 34 | 34 | 93 |
| Viable cell count (cell) | 34 | 29 | 31 | 29 | 59 |
| Viability (%) | 100 | 87.9 | 91.2 | 85.3 | 63.4 |

As shown in Table 8, the proportion of viable cells (viability) after the thawing in cryopreserving the cells using each of the hollow fiber membranes of Examples 1 to 3 was higher than that in using a commercial item or Comparative Example. These results show that the hollow fiber membranes of Examples 1 to 3 can be preferably used for the purpose of cell cryopreservation.

REFERENCE SIGNS LIST

10a: Spinning dope, 10b: Internal liquid, 11: Nozzle, 12: Guide in liquid, 13, 14, and 15: Rollers, 16: Hollow fiber membrane, 20: Aerial traveling portion, 21: Coagulation liquid

## Claims

**1.** A cell cryopreservation hollow fiber membrane, comprising a cellulose ester,
wherein breaking strength in thawing the cell cryopreservation hollow fiber membrane after freezing by vitrification freezing is 80% or more of breaking strength during wetting before the freezing.

**2.** The hollow fiber membrane according to claim 1, wherein the hollow fiber membrane comprises a nonuniform structure in a thickness direction.

**3.** The hollow fiber membrane according to claim 1 or 2, wherein an average pore size of an outer surface of the hollow fiber membrane is 1.1 or more times as large as an average pore size of an inner surface.

**4.** The hollow fiber membrane according to any one of claims 1 to 3, wherein an average area percentage in a thickness direction cross section is 40% or more and 70% or less.

**5.** The hollow fiber membrane according to any one of claims 1 to 4, wherein a variation in an area percentage of the thickness direction cross section is less than 5% in the thickness direction.

6. The hollow fiber membrane according to any one of claims 1 to 3, wherein an arithmetic average roughness of the inner surface of the hollow fiber membrane is 20 nm or less, as measured by atomic force microscopy.

FIG.1

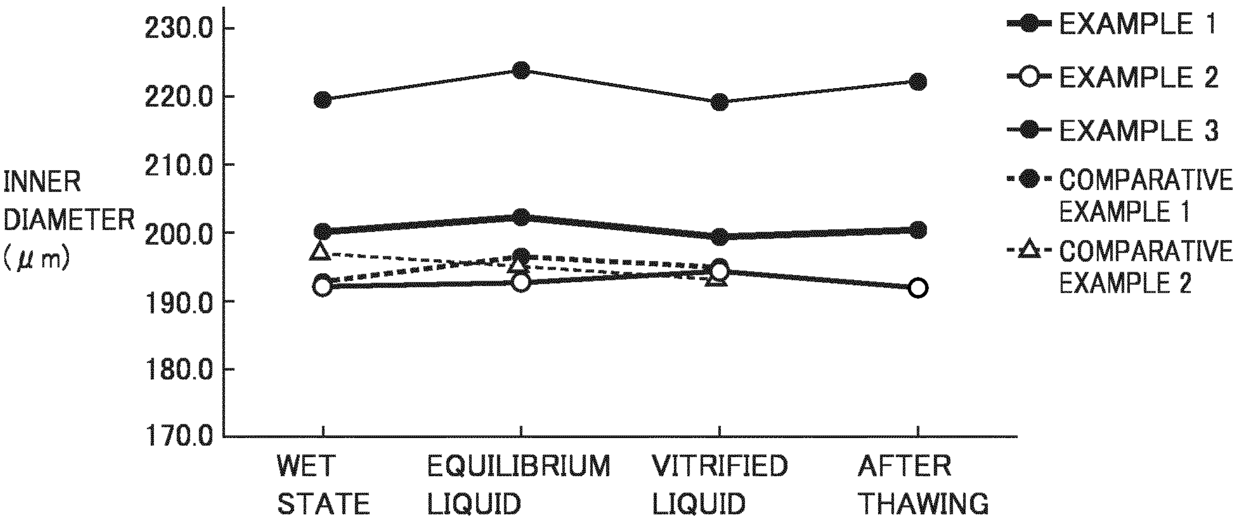
230.0
220.0
210.0
200.0
190.0
180.0
170.0
INNER DIAMETER (μm)
WET STATE
EQUILIBRIUM LIQUID
VITRIFIED LIQUID
AFTER THAWING
EXAMPLE 1
EXAMPLE 2
EXAMPLE 3
COMPARATIVE EXAMPLE 1
COMPARATIVE EXAMPLE 2

FIG.2

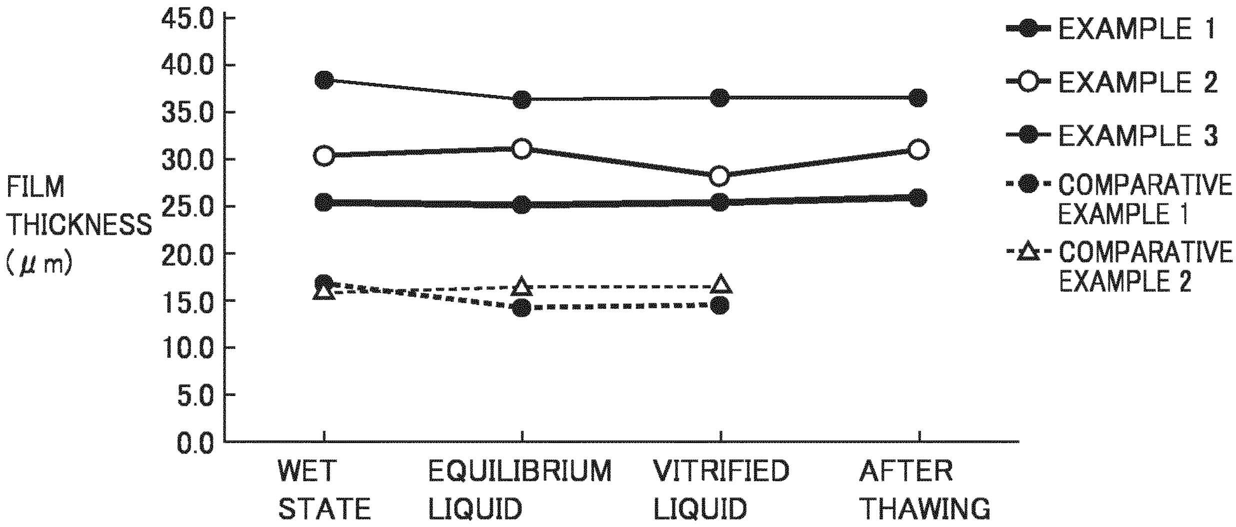
FILM THICKNESS (μm)
45.0
40.0
35.0
30.0
25.0
20.0
15.0
10.0
5.0
0.0
WET STATE
EQUILIBRIUM LIQUID
VITRIFIED LIQUID
AFTER THAWING
EXAMPLE 1
EXAMPLE 2
EXAMPLE 3
COMPARATIVE EXAMPLE 1
COMPARATIVE EXAMPLE 2

FIG.3

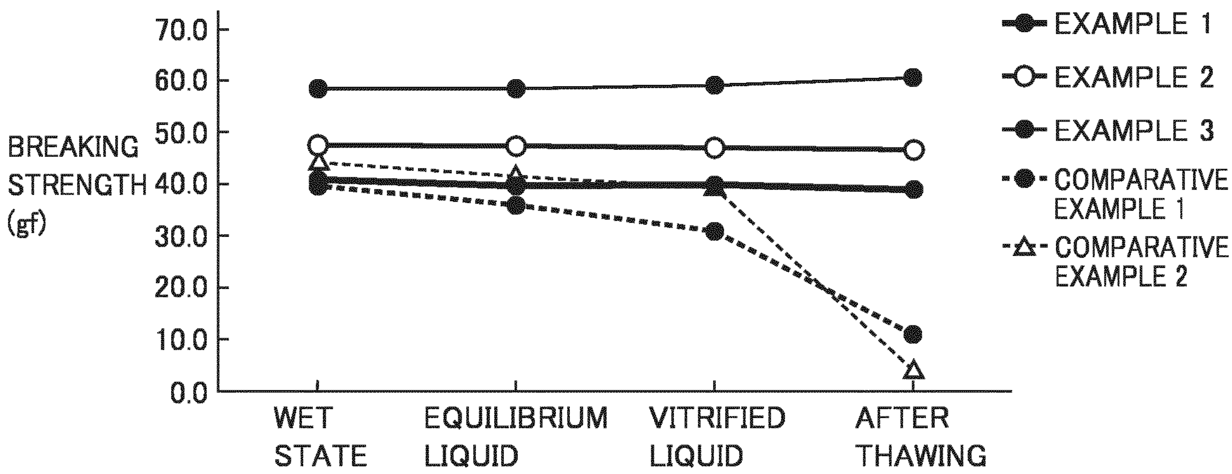
BREAKING STRENGTH (gf)
70.0
60.0
50.0
40.0
30.0
20.0
10.0
0.0
WET STATE
EQUILIBRIUM LIQUID
VITRIFIED LIQUID
AFTER THAWING
EXAMPLE 1
EXAMPLE 2
EXAMPLE 3
COMPARATIVE EXAMPLE 1
COMPARATIVE EXAMPLE 2

FIG.4

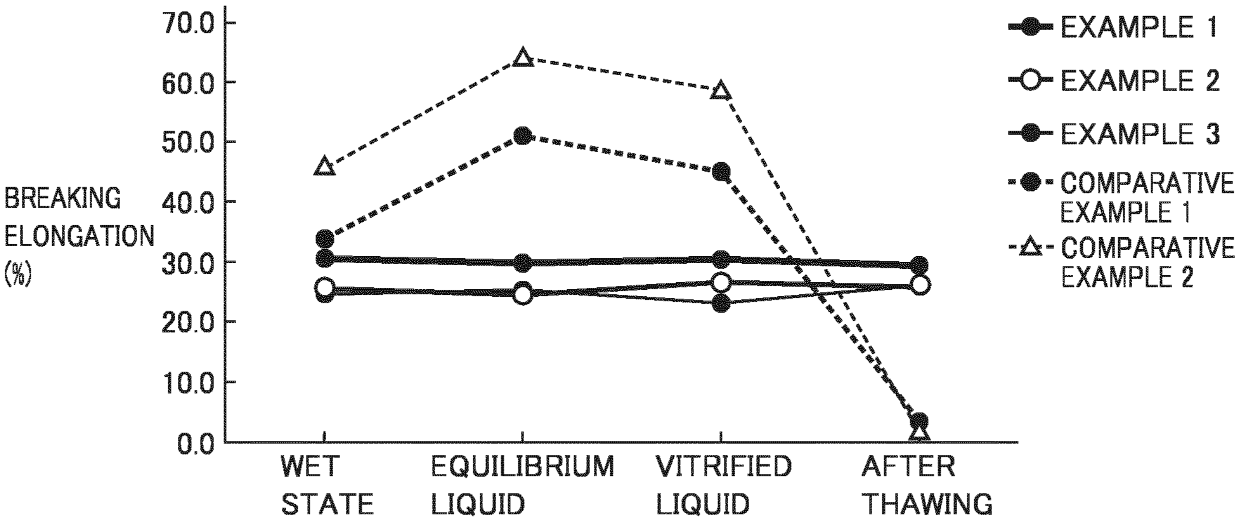
BREAKING ELONGATION (%)
70.0
60.0
50.0
40.0
30.0
20.0
10.0
0.0
WET STATE
EQUILIBRIUM LIQUID
VITRIFIED LIQUID
AFTER THAWING
EXAMPLE 1
EXAMPLE 2
EXAMPLE 3
COMPARATIVE EXAMPLE 1
COMPARATIVE EXAMPLE 2

FIG.5

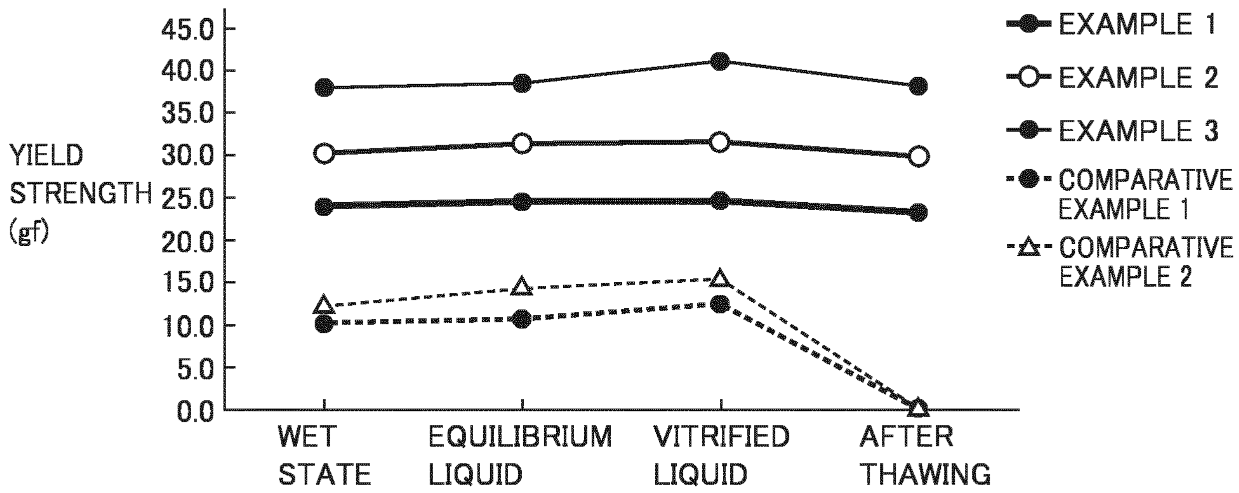
YIELD STRENGTH (gf)
45.0
40.0
35.0
30.0
25.0
20.0
15.0
10.0
5.0
0.0
WET STATE
EQUILIBRIUM LIQUID
VITRIFIED LIQUID
AFTER THAWING
EXAMPLE 1
EXAMPLE 2
EXAMPLE 3
COMPARATIVE EXAMPLE 1
COMPARATIVE EXAMPLE 2

FIG.6

SPINNING STEP

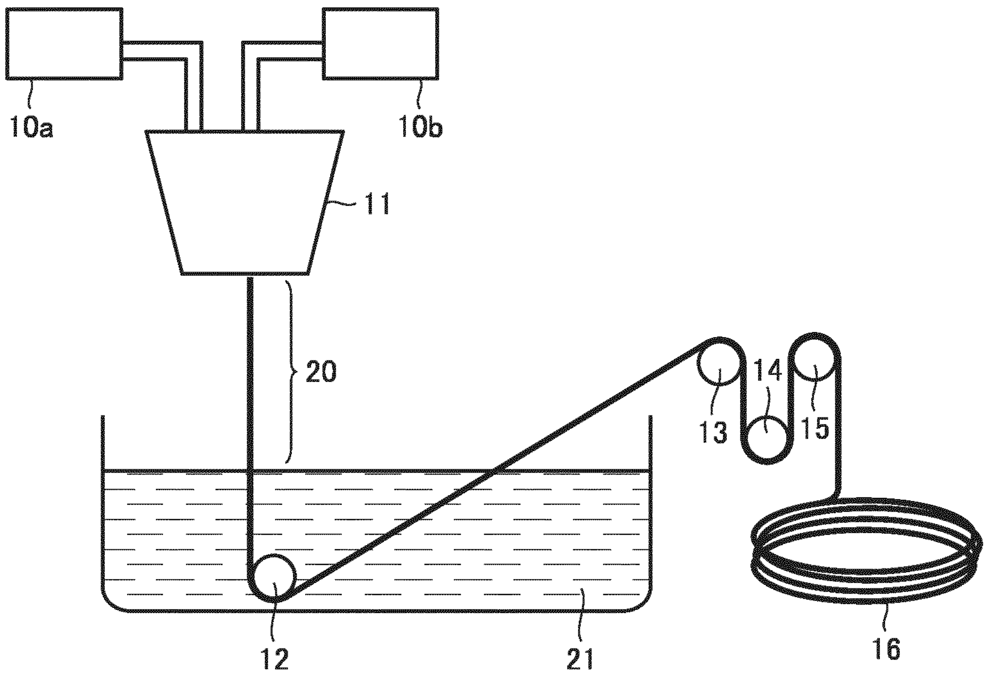

## INTERNATIONAL SEARCH REPORT

International application No.
**PCT/JP2021/039439**

**A. CLASSIFICATION OF SUBJECT MATTER**

***C12M 1/00***(2006.01)i; ***A01N 1/00***(2006.01)i; ***B01D 71/14***(2006.01)i; ***B01D 71/16***(2006.01)i; ***C12N 1/04***(2006.01)i; ***C12N 5/073***(2010.01)i; ***C12N 5/075***(2010.01)i; ***D01F 2/28***(2006.01)i
FI: C12M1/00 A; D01F2/28 A; C12N1/04; C12N5/073; C12N5/075; A01N1/00; B01D71/16; B01D71/14

According to International Patent Classification (IPC) or to both national classification and IPC

**B. FIELDS SEARCHED**

Minimum documentation searched (classification system followed by classification symbols)

C12M1/00; A01N1/00; B01D71/14; B01D71/16; C12N1/04; C12N5/073; C12N5/075; D01F2/28

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | KORNIENKO, Ekaterina V. et al. Optimization of triacetate cellulose hollow fiber vitrification (HFV) method for cryopreservation of in vitro matured bovine oocytes. Cryobiology. 07 October 2020, vol. 97, pages 66-70, doi: 10.1016/j.cryobiol.2020.10.007<br>abstract, page 67 "2.2 Oocyte vitrification", page 68 "3. Results" | 1 |
| Y | | 2-3 |
| A | | 4-6 |
| X | 内倉鮎子等, 中空糸ガラス化法の実用化に関する研究-1: 融解速度の胚生存性への影響, 第106回日本繁殖生物学会大会 講演要旨集, 2013, OR2-26, doi: 10.14882/jrds.106.0.OR2-26.0, non-official translation (UCHIKURA, Ayuko et al. Improvement of the hollow fiber vitrification method -1, Effect of melting rate on embryo viability. Proceedings of the 106th annual meeting of the Japanese Society of Animal Reproduction.)<br>entire text | 1 |
| Y | | 2-3 |
| A | | 4-6 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 January 2022** | **11 January 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2021/039439**

**C. DOCUMENTS CONSIDERED TO BE RELEVANT**

| Category* | Citation of document, with indication, where appropriate, of the relevant passages | Relevant to claim No. |
|---|---|---|
| X | KORNIENKO, E. V. et al. 32 Vitrification of in vitro-matured bovine oocytes in triacetate cellulose hollow fibres. Reproduction, Fertility and Development. 2018, vol. 31, no. 1, page 142, doi: 10.1071/RDv31n1Ab32<br>abstract | 1 |
| Y | | 2-3 |
| A | | 4-6 |
| Y | JP 2008-178814 A (TOYOBO CO., LTD.) 07 August 2008 (2008-08-07)<br>claims, paragraphs [0001], [0014], [0029], examples | 2-3 |
| A | JP 2002-306937 A (DAICEL CHEM. IND., LTD.) 22 October 2002 (2002-10-22)<br>whole document | 1-6 |

Form PCT/ISA/210 (second sheet) (January 2015)

**INTERNATIONAL SEARCH REPORT**
**Information on patent family members**

International application No.
**PCT/JP2021/039439**

| Patent document cited in search report | Publication date (day/month/year) | Patent family member(s) | Publication date (day/month/year) |
|---|---|---|---|
| JP 2008-178814 A | 07 August 2008 | (Family: none) | |
| JP 2002-306937 A | 22 October 2002 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5252556 B
- JP 5051716 B
- JP 6667903 B
- JP 5440332 B
- JP 5212837 B
- JP 2011212638 A